# EUROPEAN PATENT APPLICATION

(11) **EP 4 729 906 A1**
(43) Date of publication of application: **22.04.2026**
(21) Application number: 24825800.6
(22) Date of filing: 12.06.2024
(51) Int. Cl.: G01L 1/16, A61B 5/11, G01B 7/16, H10N 30/30, H10N 30/857

(54) **MEASURING DEVICE**

(30) Priority: 19.06.2023 JP 2023100273
(71) Applicant: Toppan Holdings Inc., Tokyo 110-0016 (JP)
(72) Inventor: SATO, Ayumu, Tokyo 110-0016 (JP); TAKASE, Satoka, Tokyo 110-0016 (JP); TAKEDA, Naoki, Tokyo 110-0016 (JP)
(74) Representative: Plasseraud IP
(86) International application number: PCT/JP2024/021381
(87) International publication number: WO 2024/262395

(57) **Abstract**

This measuring device includes: a first piezoelectric element (20) and a second piezoelectric element (21); and a piezoelectric layer 40 which is provided in common for the first piezoelectric element (20) and the second piezoelectric element (21), and which includes a first partial piezoelectric layer (40A) and a second partial piezoelectric layer (40B). The first piezoelectric element (20) includes a first lower electrode (31), the first partial piezoelectric layer (40A) provided on the first lower electrode (31), a first upper electrode (33) provided on the first partial piezoelectric layer (40A), and an expansion/contraction suppression layer (34) provided on the first partial piezoelectric layer (40A) so as to cover the first upper electrode (33). The second piezoelectric element (21) includes a second lower electrode (35), the second partial piezoelectric layer (40B) provided on the lower electrode (35), and a second upper electrode (37) provided on the second partial piezoelectric layer (40B). The modulus of elasticity of the expansion/contraction suppression layer (34) is higher than that of the piezoelectric layer (40).

## Description

### [Technical Field]

The present invention relates to a measuring device provided with a piezoelectric element.

### [Background Art]

Organic piezoelectric materials such as P(VDF/TrFE) are used in piezoelectric sensors. Because organic piezoelectric materials can be formed by coating, can be manufactured by low-temperature processes, and can also be easily formed as films, attempts are being made to apply organic piezoelectric materials to flexible sensors for wearable devices, large-area sensors, and the like. In terms of applications, various applications are being considered, such as biosensors for measuring pulse waves and mechanomyograms, tactile sensors, and pyroelectric sensors, and such applications are attracting attention.

Furthermore, displacement of the body surface at various sites, such as at the wrist or foot, is measured by attaching a piezoelectric sensor to the body. PTL 1 proposes a technique that measures the bulging of tendons in the wrist by wearing a band equipped with a plurality of piezoelectric sensors on the wrist, and uses the measurements to estimate finger movements. PTL 2 proposes a technique that similarly estimates finger movements by measuring the displacement of the body surface at the wrist using a flexible organic piezoelectric sensor, and then learning the obtained information. In addition, PTL 3 carries out precise measurements of muscle activity by attaching a flexible organic piezoelectric sensor to various sites such as the ankle or arm.

However, when measuring the displacement of the body surface with a flexible organic piezoelectric sensor, it is difficult to separate a signal originating from changes in the pressing of the sensor (contact pressure) accompanying muscle expansion and contraction, tendon bulging, and the like, from a signal originating from the bending and expansion/contraction of the sensor accompanying the bending of a joint and the associated expansion/contraction of the skin, which becomes a factor in increased signal noise and decreased reproducibility.

Among these, PTL 4 proposes a technique in which two types of piezoelectric elements are formed on a flexible substrate, and by configuring one piezoelectric element such that the center line in a thickness direction coincides with the neutral axis of the sensor, and configuring the other piezoelectric element such that the center line in the thickness direction is offset with respect to the neutral axis of the sensor, one piezoelectric element is used as a pressure sensor that does not react to bending, and the other is used as a sensor that detects bending.

However, in order to configure one sensor to coincide with the neutral axis as in PTL 4, it is necessary to make the thickness of the electrode, base material, protective film, and the like, uniform above and below the piezoelectric layer in the film thickness direction, which imposes very large design constraints, and the thinner the sensor itself is made, the more difficult control of the sensor becomes. Also, when a flexible substrate is used, even if one sensor is configured to coincide with the neutral axis, the output signal when the sensor expands and contracts in an in-plane direction cannot be suppressed, and it is difficult to separate the pressing from expansion/contraction.

### [Citation List]

### [Patent Literature]

PTL 1: JP 2005-352739 A
PTL 2: WO 2022/130684
PTL 3: JP 2020-175280 A
PTL 4: JP 2012-7911 A

### [Summary of the Invention]

### [Technical Problem]

An object of the present invention is to provide a measuring device capable of detecting expansion/contraction in an in-plane direction and expansion/contraction in a film thickness direction.

### [Solution to Problem]

According to a first aspect of the present invention, a measuring device is provided, comprising:
a first piezoelectric element and a second piezoelectric element; and
a piezoelectric layer provided in common for the first piezoelectric element and the second piezoelectric element, and which includes a first partial piezoelectric layer and a second partial piezoelectric layer, wherein
the first piezoelectric element includes
a first lower electrode,
the first partial piezoelectric layer provided on the first lower electrode,
a first upper electrode provided on the first partial piezoelectric layer, and
an expansion/contraction suppression layer provided on the first partial piezoelectric layer so as to cover the first upper electrode,
the second piezoelectric element includes
a second lower electrode,
the second partial piezoelectric layer provided on the second lower electrode, and
a second upper electrode provided on the second partial piezoelectric layer, and
the expansion/contraction suppression layer has a higher modulus of elasticity than the piezoelectric layer.

According to a second aspect of the present invention, the measuring device according to the first aspect is provided, further comprising a first protective layer provided on the piezoelectric layer so as to cover the expansion/contraction suppression layer and the second upper electrode.

According to a third aspect of the present invention, a measuring device is provided, comprising: a first piezoelectric element and a second piezoelectric element; and a piezoelectric layer provided in common for the first piezoelectric element and the second piezoelectric element, and which includes a first partial piezoelectric layer and a second partial piezoelectric layer, wherein the first piezoelectric element includes a first lower electrode, the first partial piezoelectric layer provided on the first lower electrode, a first upper electrode provided on the first partial piezoelectric layer, a first protective layer provided on the first partial piezoelectric layer so as to cover the first upper electrode, and an expansion/contraction suppression layer provided on the first protective layer, the second piezoelectric element includes a second lower electrode, the second partial piezoelectric layer provided on the second lower electrode, and a second upper electrode provided on the second partial piezoelectric layer, the first protective layer is formed so as to further cover the second upper electrode, and the expansion/contraction suppression layer has a higher modulus of elasticity than the piezoelectric layer.

According to a fourth aspect of the present invention, the measuring device according to the first or third aspect is provided, wherein the piezoelectric layer has flexibility or an expansion/contraction property.

According to a fifth aspect of the present invention, the measuring device according to the first or third aspect is provided, wherein the piezoelectric layer is formed of an organic piezoelectric material having an expansion/contraction property.

According to a sixth aspect of the present invention, the measuring device according to the first or third aspect is provided, further comprising a second protective layer provided on a bottom surface of the piezoelectric layer so as to cover the first lower electrode and the second lower electrode.

According to a seventh aspect of the present invention, a measuring device is provided, comprising: a substrate; a first piezoelectric element and a second piezoelectric element provided on the substrate; and a protective layer covering the first piezoelectric element and the second piezoelectric element, wherein the first piezoelectric element includes a first lower electrode provided on the substrate, a first piezoelectric layer provided on the first lower electrode, a first upper electrode provided on the first piezoelectric layer, and an expansion/contraction suppression layer provided on the substrate so as to cover the first upper electrode, the first piezoelectric layer, and the first upper electrode, the second piezoelectric element includes a second lower electrode provided on the substrate, a second piezoelectric layer provided on the second lower electrode, and a second upper electrode provided on the second piezoelectric layer, and the expansion/contraction suppression layer has a higher modulus of elasticity than the first piezoelectric layer and the protective layer.

According to an eighth aspect of the present invention, the measuring device according to the seventh aspect is provided, wherein the substrate has flexibility or an expansion/contraction property.

According to a ninth aspect of the present invention, the measuring device according to the seventh aspect is provided, wherein the first piezoelectric layer and the second piezoelectric layer are formed of an organic piezoelectric material having an expansion/contraction property.

According to a tenth aspect of the present invention, the measuring device according to the first, third, or seventh aspect is provided, wherein a plurality of the first piezoelectric elements and second piezoelectric elements are arranged in an array.

According to an eleventh aspect of the present invention, the measuring device according to the first, third, or seventh aspect is provided, further comprising a control unit that calculates an output voltage ratio between the first piezoelectric element and the second piezoelectric element, and based on the output voltage ratio, determines a degree of influence indicating whether there is expansion/contraction in an in-plane direction or expansion/contraction in a film thickness direction.

According to a twelfth aspect of the present invention, the measuring device according to the first, third, or seventh aspect is provided, further comprising a control unit that calculates an output voltage ratio between the first piezoelectric element and the second piezoelectric element, and based on a comparison result between the output voltage ratio and a threshold value, determines whether there is expansion/contraction in an in-plane direction or expansion/contraction in a film thickness direction.

### [Advantageous Effects of the Invention]

According to the present invention, it is possible to provide a measuring device capable of detecting expansion/contraction in an in-plane direction and expansion/contraction in a film thickness direction.

### [Brief Description of the Drawings]

Fig. 1 is a block diagram of a measuring device according to a first embodiment of the present invention.
Fig. 2 is a plan view of a piezoelectric element array.
Fig. 3 is a cross-sectional view of a first piezoelectric element and a second piezoelectric element constituting a single unit piezoelectric element.
Fig. 4 is a diagram describing a definition relating to the polarization direction of a piezoelectric layer.
Fig. 5 is a diagram describing a compression operation in the film thickness direction of a piezoelectric layer.
Fig. 6 is a diagram describing an expansion operation in the in-plane direction of a piezoelectric layer.
Fig. 7 is a diagram describing a state where the piezoelectric element array is expanded in the in-plane direction.
Fig. 8 is a diagram describing the output voltages of the first piezoelectric element and the second piezoelectric element during expansion/contraction in the in-plane direction.
Fig. 9 is a diagram describing the output voltages of the first piezoelectric element and the second piezoelectric element during expansion/contraction in the film thickness direction.
Fig. 10 is a diagram describing an output voltage ratio between the first piezoelectric element and the second piezoelectric element.
Fig. 11 is a flowchart describing a measurement operation of the measuring device.
Fig. 12 is a diagram describing an operation that determines a degree of influence relating to expansion/contraction.
Fig. 13 is a flowchart describing a measurement operation of a measuring device according to a second embodiment of the present invention.
Fig. 14 is a cross-sectional view of a first piezoelectric element and a second piezoelectric element constituting a single unit piezoelectric element according to a third embodiment of the present invention.
Fig. 15 is a cross-sectional view of a first piezoelectric element and a second piezoelectric element according to a modification.
Fig. 16 is a plan view of a piezoelectric element array according to a fourth embodiment of the present invention.
Fig. 17 is a cross-sectional view of a first piezoelectric element and a second piezoelectric element constituting a single unit piezoelectric element.
Fig. 18 is a diagram describing a state where the piezoelectric element array is expanded in the in-plane direction.
Fig. 19 is a plan view of a piezoelectric element array according to a fifth embodiment of the present invention.

### [Description of the Embodiments]

Hereinafter, embodiments will be described with reference to the drawings. However, the drawings are schematic or conceptual, and the dimensions, ratios, and the like in the drawings are not necessarily identical to the actual values. Furthermore, when the same part is illustrated in different drawings, the relationships between the dimensions and ratios may be depicted differently from each other. In particular, some of the embodiments described below exemplify devices and methods for embodying the technical ideas of the present invention, and the technical idea of the present invention is not specified by shapes, structures, arrangements, and the like of the components. In the following description, elements having the same function and configuration are denoted by the same reference signs, and duplicate descriptions are omitted.

### [1] First Embodiment

### [1-1] Configuration of Measuring Device 1

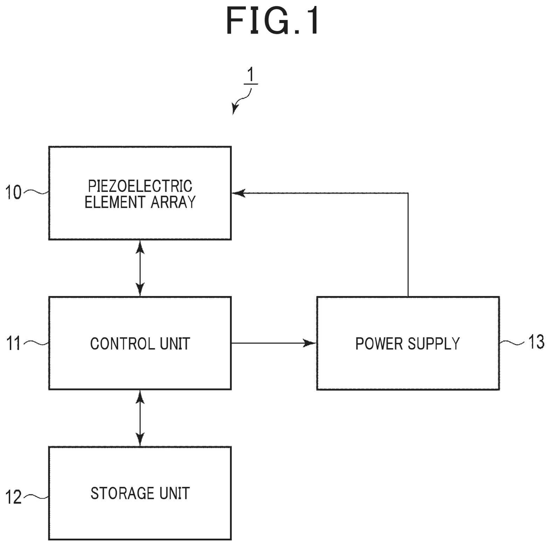

Fig. 1 is a block diagram of a measuring device 1 according to a first embodiment of the present invention. The measuring device 1 is a sensor device that utilizes the piezoelectric effect, and is capable of outputting an electrical signal according to the pressure applied to a piezoelectric body. The measuring device 1 includes a piezoelectric element array 10, a control unit 11, a storage unit 12, and a power supply 13.

The piezoelectric element array 10 includes a plurality of piezoelectric elements. The piezoelectric element array 10 is a measuring unit that detects and measures the pressure applied to the piezoelectric body. Furthermore, the piezoelectric element array 10 is a measuring unit capable of detecting and measuring expansion/contraction in an in-plane direction and expansion/contraction in a film thickness direction.

The control unit 11 is constituted by one or more processors such as a CPU (Central Processing Unit) or an MPU (Micro Processing Unit). The control unit 11 realizes various functions by executing programs stored in the storage unit 12. The control unit 11 receives output voltages from the piezoelectric elements, and executes arithmetic processing based on the output voltages. Furthermore, the control unit 11 executes processing to determine a degree of influence relating to the expansion/contraction of the piezoelectric element array 10.

The storage unit 12 includes a non-volatile storage device such as a ROM (Read Only Memory), and a volatile storage device such as a RAM (Random Access Memory) and registers. The storage unit 12 stores programs executed by the control unit 11. The storage unit 12 stores various data necessary for the control by the control unit 11.

The power supply 13, based on the control by the control unit 11, generates various voltages necessary for the operation of each circuit within the measuring device 1, and supplies the voltages to the corresponding circuits. The power supply 13 also supplies various voltages to the piezoelectric element array 10.

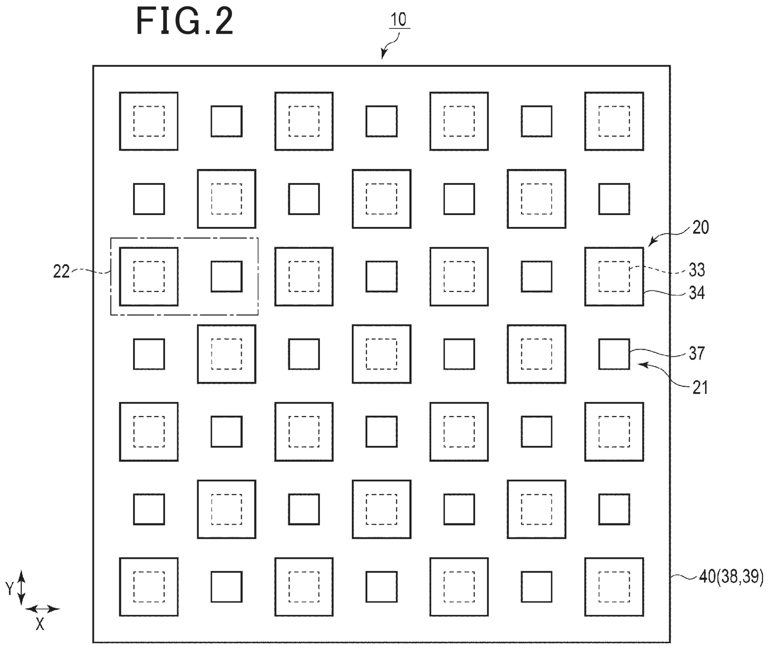

Fig. 2 is a plan view of the piezoelectric element array 10. In Fig. 2, the X direction is a direction along one side of the piezoelectric element array 10, and the Y direction is a direction orthogonal to the X direction.

The piezoelectric element array 10 includes a plurality of first piezoelectric elements 20 and a plurality of second piezoelectric elements 21. The plurality of first piezoelectric elements 20 and the plurality of second piezoelectric elements 21 are arranged in a matrix overall. An adjacent first piezoelectric element 20 and second piezoelectric element 21 constitute a unit piezoelectric element 22.

The plurality of first piezoelectric elements 20 and the plurality of second piezoelectric elements 21 arranged in the X direction are alternately arranged. The plurality of first piezoelectric elements 20 and the plurality of second piezoelectric elements 21 arranged in the Y direction are alternately arranged. In other words, every other element arranged in the X direction and Y direction is one of the plurality of first piezoelectric elements 20. Every other element arranged in the X direction and Y direction is one of the plurality of second piezoelectric elements 21. The plurality of first piezoelectric elements 20 are arranged so as to be adjacent in the diagonal directions. The plurality of second piezoelectric elements 21 are arranged so as to be adjacent in the diagonal directions. In other words, the plurality of first piezoelectric elements 20 are arranged in a staggered pattern, and the plurality of second piezoelectric elements 21 are arranged in a staggered pattern.

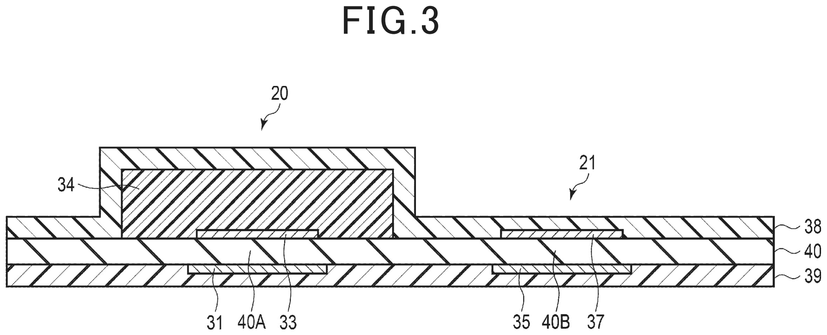

Fig. 3 is a cross-sectional view of a first piezoelectric element 20 and a second piezoelectric element 21 constituting a single unit piezoelectric element 22. Fig. 3 is a cross-sectional view of the unit piezoelectric element 22 of Fig. 2 taken along the X direction.

The piezoelectric element array 10 includes a piezoelectric layer 40. The piezoelectric layer 40 is provided in common for the plurality of first piezoelectric elements 20 and the plurality of second piezoelectric elements 21. The piezoelectric layer 40 is provided over the entire piezoelectric element array 10. The piezoelectric layer 40 also functions as a substrate of the piezoelectric element array 10. The piezoelectric layer 40 has flexibility and/or an expansion/contraction property.

The piezoelectric layer 40 is composed of, for example, an organic piezoelectric material. The piezoelectric layer 40 is composed of, for example, a polymeric piezoelectric material, such as polyvinylidene fluoride (PVDF), polyvinylidene fluoride/trifluoroethylene copolymer (P(VDF/TrFE)), or vinylidene cyanide/vinyl acetate copolymer (P(VDCN/VAc)). Also, in order to impart an expansion/contraction property to P(VDF/TrFE), an acrylic resin (PMMA: polymethyl methacrylate) may be added to P(VDF/TrFE). The thickness of the piezoelectric layer 40 is, for example, about 40 µm.

The first piezoelectric element 20 includes a first lower electrode 31, a partial piezoelectric layer 40A, a first upper electrode 33, and an expansion/contraction suppression layer 34. The partial piezoelectric layer 40A is included in the piezoelectric layer 40, and corresponds to a region of the piezoelectric layer 40 that functions as a single first piezoelectric element 20.

The first lower electrode 31 is provided on the bottom surface of the partial piezoelectric layer 40A. The first lower electrode 31 may have, for example, a square shape. The first lower electrode 31 is composed of a metal material or an organic conductive material. Examples of the metal material include silver (Ag), silver paste, molybdenum (Mo), aluminum (Al), and chromium (Cr). Examples of the organic conductive material include PEDOT/PSS and carbon paste. PEDOT/PSS is a composite of poly(3,4-ethylenedioxythiophene) (PEDOT) and polystyrene sulfonic acid (PSS). The thickness of the first lower electrode 31 is, for example, about 10 µm.

The first upper electrode 33 is provided on the upper surface of the partial piezoelectric layer 40A. The first upper electrode 33 may have, for example, a square shape. Like the first lower electrode 31, the first upper electrode 33 is composed of a metal material or an organic conductive material. For example, the area of the first upper electrode 33 may be smaller than the area of the first lower electrode 31. The area of the first upper electrode 33 may be approximately the same as that of the first lower electrode 31, or may be larger. The thickness of the first upper electrode 33 is, for example, about 10 µm.

An expansion/contraction suppression layer 34 is provided on the upper surface of the partial piezoelectric layer 40A so as to cover the first upper electrode 33. The expansion/contraction suppression layer 34 is composed of a material with a higher modulus of elasticity than the piezoelectric layer 40. Furthermore, the expansion/contraction suppression layer 34 is composed of a material with a higher modulus of elasticity than a protective layer 38, which will be described later. The modulus of elasticity is the ratio of stress to strain (stress / strain) in an elastic deformation when a force is applied to an elastic body.

The expansion/contraction suppression layer 34 has a function of suppressing the expansion/contraction of the piezoelectric layer 40 due to stress, and in particular, has a function of suppressing the expansion/contraction of the piezoelectric layer 40 in the in-plane direction. The expansion/contraction suppression layer 34 is preferably an insulating material, but a conductive material may also be used. As the expansion/contraction suppression layer 34, for example, a cyanoacrylate resin is used. The thickness of the expansion/contraction suppression layer 34 is, for example, about 600 µm. The expansion/contraction suppression layer 34 is formed by coating on the piezoelectric layer 40 and the first upper electrode 33. The expansion/contraction suppression layer 34 may also be formed by being bonded to the piezoelectric layer 40 and the first upper electrode 33 using an adhesive.

The second piezoelectric element 21 includes a second lower electrode 35, a partial piezoelectric layer 40B, and a second upper electrode 37. The partial piezoelectric layer 40B is included in the piezoelectric layer 40, and corresponds to a region of the piezoelectric layer 40 that functions as a single second piezoelectric element 21.

A second lower electrode 35 is provided on the bottom surface of the partial piezoelectric layer 40B. A second upper electrode 37 is provided on the upper surface of the partial piezoelectric layer 40B. The configuration of the second lower electrode 35 and the second upper electrode 37 is the same as that of the first lower electrode 31 and the first upper electrode 33. The areas and thicknesses of the second lower electrode 35 and the second upper electrode 37 are also the same as those of the first lower electrode 31 and the first upper electrode 33. Note that the materials, areas, and thicknesses of the second lower electrode 35 and the second upper electrode 37 may be different from those of the first lower electrode 31 and the first upper electrode 33.

On the piezoelectric layer 40, a protective layer 38 is provided so as to cover the expansion/contraction suppression layer 34 and the second upper electrode 37. The protective layer 38 is provided over the entire piezoelectric element array 10. The protective layer 38 is composed of an insulating material, and is composed of a resin having flexibility and/or an expansion/contraction property. It is desirable that the protective layer 38 is a resin with high moisture permeability. The protective layer 38 is composed of, for example, a polyurethane resin, a polyolefin resin, a polystyrene resin, or a polyester resin. The thickness of the protective layer 38 is, for example, about 50 µm. The protective layer 38 is formed by coating or by bonding using an adhesive.

On the bottom surface of the piezoelectric layer 40, a protective layer 39 is provided so as to cover the first lower electrode 31 and the second lower electrode 35. The protective layer 39 is provided over the entire piezoelectric element array 10. The material of the protective layer 39 is the same as that of the protective layer 38. The thickness of the protective layer 39 is, for example, about 50 µm. The protective layer 39 is formed by coating or by bonding using an adhesive.

The lower electrodes and upper electrodes of the first piezoelectric element 20 and the second piezoelectric element 21 are respectively drawn out to the outside of the piezoelectric element array 10 using lead wires (not shown), and are wired to the control unit 11 and the power supply 13.

### [1-2] Operation

Next, the operation of the measuring device 1 configured as described above will be described.

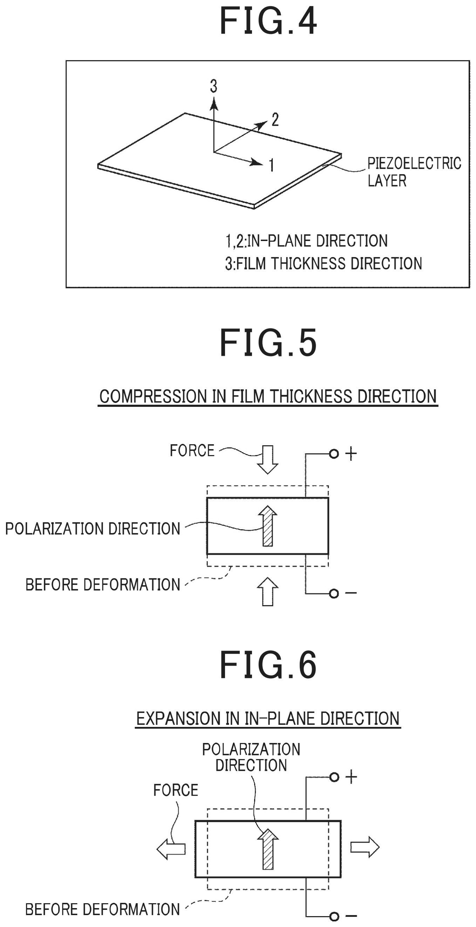

First, the characteristics of the piezoelectric layer will be described. The piezoelectric layer is composed of P(VDF/TrFE). Fig. 4 is a diagram describing a definition relating to the polarization direction of a piezoelectric layer. An arbitrary direction in the plane of the piezoelectric layer is defined as direction "1". A direction orthogonal to direction "1" in the plane of the piezoelectric layer is defined as direction "2". Both direction "1" and direction "2" are in-plane directions. A direction orthogonal to the plane of the piezoelectric layer is defined as direction "3". Direction "3" is the film thickness direction.

Fig. 5 is a diagram describing a compression operation in the film thickness direction of a piezoelectric layer. Fig. 5 shows a state where a force is applied to the piezoelectric layer in the film thickness direction, and the piezoelectric layer is compressed in the film thickness direction. The rectangle in Fig. 5 illustrated with a dashed line represents the piezoelectric layer before deformation, and the rectangle illustrated with a solid line represents the piezoelectric layer after deformation.

When the piezoelectric layer is compressed in the film thickness direction, the polarization direction of the piezoelectric layer is in the upward direction in the drawing (direction "3"). A positive charge is generated on the upper side of the piezoelectric layer. When the piezoelectric layer is compressed in the film thickness direction, the piezoelectric strain constant is d₃₃. The left digit of the piezoelectric strain constant indicates the polarization direction, and the right digit indicates the deformation direction. A compression operation in the film thickness direction is also referred to as a d₃₃ mode.

Fig. 6 is a diagram describing an expansion operation (stretching operation) in the in-plane direction of a piezoelectric layer. Fig. 6 shows a state where the piezoelectric layer is pulled in the in-plane direction, and the piezoelectric layer is expanded in the in-plane direction. The rectangle in Fig. 6 illustrated with a dashed line represents the piezoelectric layer before deformation, and the rectangle illustrated with a solid line represents the piezoelectric layer after deformation.

When the piezoelectric layer is expanded in the in-plane direction, the polarization direction of the piezoelectric layer is in the upward direction in the drawing (direction "3"). A positive charge is generated on the upper side of the piezoelectric layer. When the piezoelectric layer is expanded in the in-plane direction, the piezoelectric strain constant is d₃₁ or d₃₂. An expansion operation in the in-plane direction is also referred to as a d₃₁ mode or a d₃₂ mode.

As shown in Figs. 5 and 6, in the piezoelectric layer, charges of the same polarity are generated in the compression operation in the film thickness direction and in the expansion operation in the in-plane direction. That is, the first piezoelectric element 20 and the second piezoelectric element 21 each output a voltage of the same polarity in the compression operation in the film thickness direction and in the expansion operation in the in-plane direction.

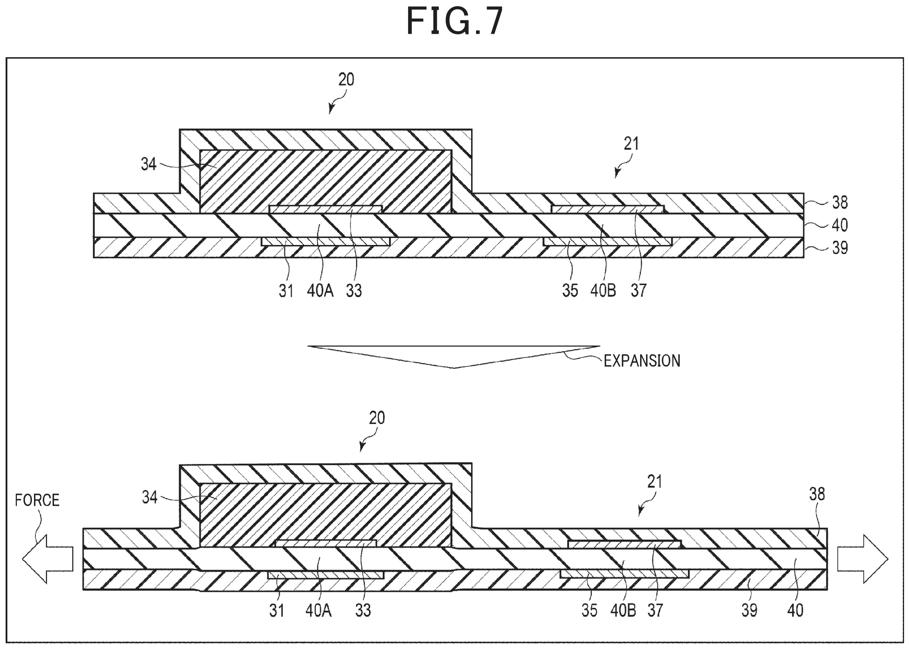

Fig. 7 is a diagram describing a state where the piezoelectric element array 10 is expanded in the in-plane direction. Fig. 7 shows a single unit piezoelectric element 22 (the first piezoelectric element 20 and the second piezoelectric element 21) extracted for illustration. For example, when bending occurs in the piezoelectric element array 10, the piezoelectric element array 10 is expanded in the in-plane direction.

The piezoelectric layer 40, the protective layer 38, and the protective layer 39 have flexibility and/or an expansion/contraction property. When bending occurs in the piezoelectric element array 10, the partial piezoelectric layer 40B of the second piezoelectric element 21 is expanded in the in-plane direction, and stress is applied in the expansion direction.

On the other hand, the first piezoelectric element 20 is provided with the expansion/contraction suppression layer 34, which has a high modulus of elasticity. Therefore, in the partial piezoelectric layer 40A of the first piezoelectric element 20, compared to the partial piezoelectric layer 40B of the second piezoelectric element 21, because the modulus of elasticity of the expansion/contraction suppression layer 34 is high, a large stress is applied from the expansion/contraction suppression layer 34 to the partial piezoelectric layer 40A during expansion/contraction in the in-plane direction. As a result, the first piezoelectric element 20 has a larger output voltage than the second piezoelectric element 21.

As a method of detecting the output voltages of the first piezoelectric element 20 and the second piezoelectric element 21, the potential of one of a lower electrode and an upper electrode of the piezoelectric element is used as a reference (for example, a ground voltage (0 V)), and the difference between the reference and the potential of the other electrode is detected.

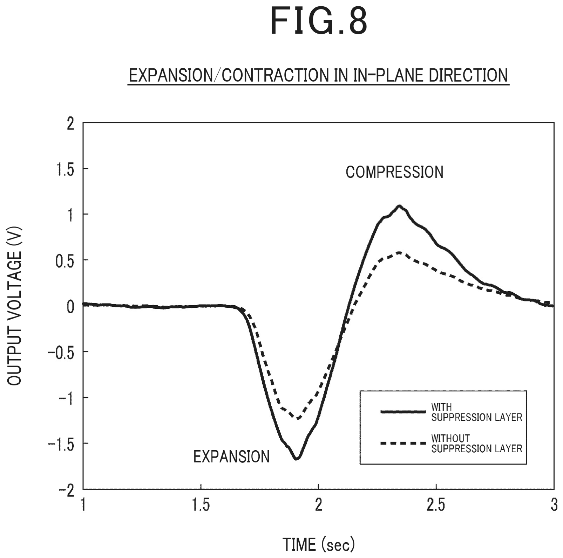

Fig. 8 is a diagram describing the output voltages of the first piezoelectric element 20 and the second piezoelectric element 21 during expansion/contraction in the in-plane direction. The vertical axis of Fig. 8 represents the output voltage (V), and the horizontal axis represents time (sec). The solid line in Fig. 8 indicates the first piezoelectric element 20 (with suppression layer), and the dashed line indicates the second piezoelectric element 21 (without suppression layer). The suppression layer refers to the expansion/contraction suppression layer. In the experiment of Fig. 8, wiring is connected to the piezoelectric elements such that the output voltage becomes a negative voltage during expansion (pulling). In the experiment of Fig. 8, the thickness of the expansion/contraction suppression layer 34 is about 670 µm. The expansion in Fig. 8 is a case where the piezoelectric element is expanded in the in-plane direction, and the compression in Fig. 8 is a case where the piezoelectric element is compressed in the in-plane direction.

As can be understood from Fig. 8, when the piezoelectric element is expanded in the in-plane direction, the first piezoelectric element 20 (with suppression layer) has a greater output voltage than the second piezoelectric element 21 (without suppression layer). Here, "greater output voltage" means that the absolute value of the voltage is greater. Furthermore, when the piezoelectric element is compressed in the in-plane direction, the first piezoelectric element 20 has a greater output voltage than the second piezoelectric element 21.

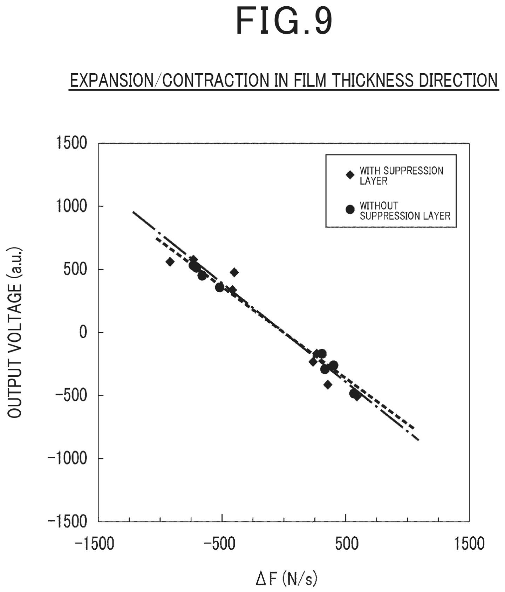

Fig. 9 is a diagram describing the output voltages of the first piezoelectric element 20 and the second piezoelectric element 21 during expansion/contraction in the film thickness direction. The vertical axis of Fig. 9 represents the output voltage (arbitrary unit), and the horizontal axis represents the rate of change of force ΔF (N/s). In the experiment of Fig. 9, wiring is connected to the piezoelectric elements such that the output voltage becomes a negative voltage during compression. In the experiment of Fig. 9, the thickness of the expansion/contraction suppression layer 34 is about 670 µm. In Fig. 9, the region where the output voltage is negative represents an operation where the piezoelectric element is compressed in the film thickness direction, and the region where the output voltage is positive represents an operation where the piezoelectric element is expanded in the film thickness direction.

In the graph of the first piezoelectric element 20 (with suppression layer), the regression line is represented by "y = -0.7253x", and the coefficient of determination is represented by "R² = 0.9898". In the graph of the second piezoelectric element 21 (without suppression layer), the regression line is represented by "y = -0.7829x", and the coefficient of determination is represented by "R² = 0.9477". As can be understood from Fig. 9, when the piezoelectric element is compressed and expanded in the film thickness direction, there is almost no difference in the output voltage between the first piezoelectric element 20 and the second piezoelectric element 21.

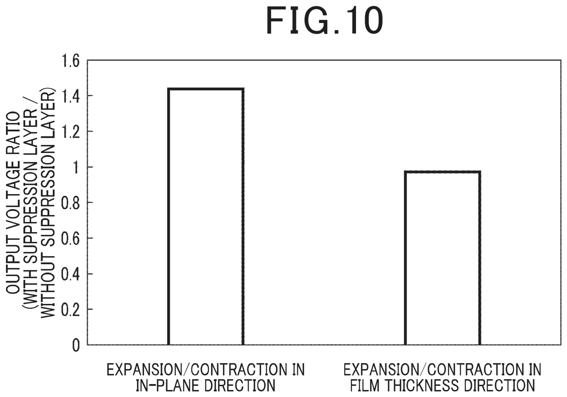

Fig. 10 is a diagram describing an output voltage ratio between the first piezoelectric element 20 and the second piezoelectric element 21. Fig. 10 shows a graph for expansion/contraction of the piezoelectric element in the in-plane direction, and a graph for expansion/contraction of the piezoelectric element in the film thickness direction. The vertical axis of Fig. 10 is the output voltage ratio (with suppression layer / without suppression layer), that is, the ratio of the output voltage of the first piezoelectric element 20 (with suppression layer) to the output voltage of the second piezoelectric element 21 (without suppression layer).

The output voltage ratio is obtained by detecting the first output voltage of the first piezoelectric element 20 and the second output voltage of the second piezoelectric element 21, and then performing the calculation "first output voltage / second output voltage".

As can be understood from Fig. 10, the output voltage ratio during expansion/contraction in the in-plane direction is relatively large, and the output voltage ratio during expansion/contraction in the film thickness direction is relatively small. Specifically, the output voltage ratio during expansion/contraction in the in-plane direction is about 1.4, and the output voltage ratio during expansion/contraction in the film thickness direction is about 1.0. Therefore, the closer the output voltage ratio is to 1.4, the stronger the influence of expansion/contraction in the in-plane direction, and it can be determined that, for example, bending or pulling is occurring in the piezoelectric element array 10. The closer the output voltage ratio is to 1.0, the stronger the influence of expansion/contraction in the film thickness direction, and it can be determined that, for example, the piezoelectric element array 10 is being pressed. In the present specification, pressing means that the piezoelectric element is being compressed in the film thickness direction. Note that the minimum unit of resolution for pressing is determined by the size of a single set of the first piezoelectric element 20 and the second piezoelectric element 21.

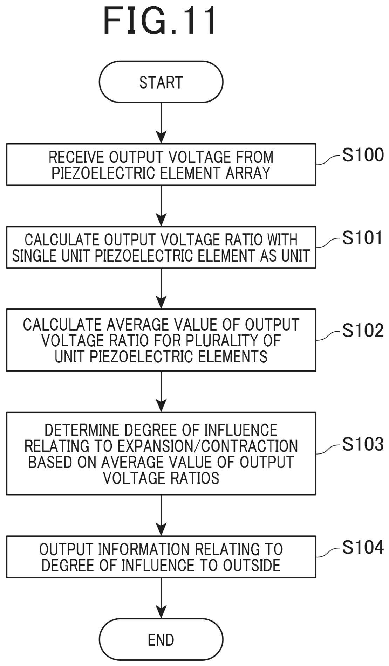

Next, the operation of determining expansion/contraction in the in-plane direction and expansion/contraction in the film thickness direction will be described. Fig. 11 is a flowchart describing a measurement operation of the measuring device 1.

The control unit 11 receives output voltages from the piezoelectric element array 10 (step S100). That is, the control unit 11 receives output voltages from the plurality of first piezoelectric elements 20 and the plurality of second piezoelectric elements 21.

Then, the control unit 11 calculates the output voltage ratio with a single unit piezoelectric element 22 (the first piezoelectric element 20 and the second piezoelectric element 21) as a unit (step S101). That is, the control unit 11 calculates "(first output voltage of first piezoelectric element 20) / (second output voltage of second piezoelectric element 21)" with a single unit piezoelectric element 22 as a unit.

Then, the control unit 11 calculates the average value of the output voltage ratios of the plurality of unit piezoelectric elements 22 (step S102).

Next, the control unit 11, based on the average value of the output voltage ratios calculated in step S102, determines the degree of influence relating to expansion/contraction (step S103).

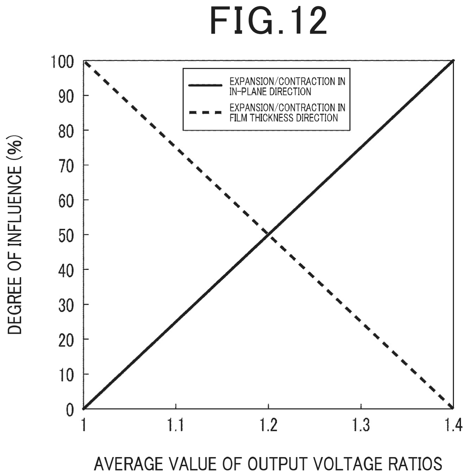

Fig. 12 is a diagram describing an operation that determines a degree of influence relating to expansion/contraction. The horizontal axis of Fig. 12 represents the average value of the output voltage ratio, and the vertical axis represents the degree of influence relating to expansion/contraction (%). The degree of influence relating to expansion/contraction is represented by the degree of influence relating to expansion/contraction in the in-plane direction and the degree of influence relating to expansion/contraction in the film thickness direction. Specifically, the degree of influence relating to expansion/contraction in the in-plane direction is the degree of influence relating to bending or pulling, and the degree of influence relating to expansion/contraction in the film thickness direction is the degree of influence relating to pressing.

In the present embodiment, as an example, based on the experimental results of Fig. 10, the average value of the output voltage ratio is set in the range of 1.0 or more and 1.4 or less. The degree of influence relating to expansion/contraction in the in-plane direction changes from 0% to 100%. The degree of influence relating to expansion/contraction in the in-plane direction and the average value of the output voltage ratio are in a proportional relationship. The degree of influence relating expansion/contraction in the film thickness direction changes from 100% to 0%. The degree of influence relating to expansion/contraction in the film thickness direction and the average value of the output voltage ratio are in an inversely proportional relationship.

Specifically, when the average value of the output voltage ratio is 1.0, the control unit 11 determines that the degree of influence relating to expansion/contraction in the in-plane direction is 0%, and the degree of influence relating to expansion/contraction in the film thickness direction is 100%. When the average value of the output voltage ratio is 1.1, the control unit 11 determines that the degree of influence relating to expansion/contraction in the in-plane direction is 25% and the degree of influence relating to expansion/contraction in the film thickness direction is 75%. When the average value of the output voltage ratio is 1.2, the control unit 11 determines that the degree of influence relating to expansion/contraction in the in-plane direction is 50% and the degree of influence relating to expansion/contraction in the film thickness direction is 50%. When the average value of the output voltage ratio is 1.3, the control unit 11 determines that the degree of influence relating to expansion/contraction in the in-plane direction is 75% and the degree of influence relating to expansion/contraction in the film thickness direction is 25%. When the average value of the output voltage ratio is 1.4, the control unit 11 determines that the degree of influence relating to expansion/contraction in the in-plane direction is 100% and the degree of influence relating to expansion/contraction in the film thickness direction is 0%.

Note that the piezoelectric element array 10 may be divided into a plurality of regions, and the degree of influence may be determined for each divided region. Furthermore, the degree of influence may be determined for each unit piezoelectric element 22. The determination processing may be performed by the control unit 11 disposed inside the measuring device 1, or may be performed by a control unit 11 disposed outside the measuring device 1 and connected in a wired or wireless fashion.

Then, the control unit 11 outputs information relating to the degree of influence determined in step S103 to the outside (step S104).

The measuring device 1 can be applied to a wearable device used when attached to the wrist, arm, or the like. By wearing the measuring device 1 on the body, it is possible to measure the displacement of the body surface.

### [1-3] Effects of First Embodiment

According to the first embodiment, for example, when a force is applied to the piezoelectric element array 10 according to the displacement of the body surface on which the measuring device 1 is worn, it is possible to perform a determination by distinguishing between expansion/contraction in the in-plane direction (including expansion/contraction due to bending or pulling) and expansion/contraction in the film thickness direction (including expansion/contraction due to pressing). As a result, it is possible to realize the measuring device 1 capable of detecting expansion/contraction in the in-plane direction and expansion/contraction in the film thickness direction.

Specifically, it is possible to perform a detection by distinguishing between pressing (contact pressure) of the piezoelectric element array 10 accompanying muscle expansion and contraction, tendon bulging, and the like, and bending and pulling of the piezoelectric element array 10 accompanying the bending of a joint and the associated expansion/contraction of the skin. This makes it possible to perform high-precision measurement of body surface displacement.

Furthermore, because the piezoelectric element array 10 is configured using a material having flexibility and/or an expansion/contraction property, the piezoelectric element array 10 is more easily deformed. Therefore, since the piezoelectric element array 10 can be deformed according to the displacement of the body surface, it becomes possible to perform high-precision measurement of body surface displacement.

### [2] Second Embodiment

The second embodiment is another example of a determination operation relating to expansion/contraction. In the second embodiment, it is determined whether the force applied to the piezoelectric element array 10 is expansion/contraction due to bending or pulling, or expansion/contraction due to pressing.

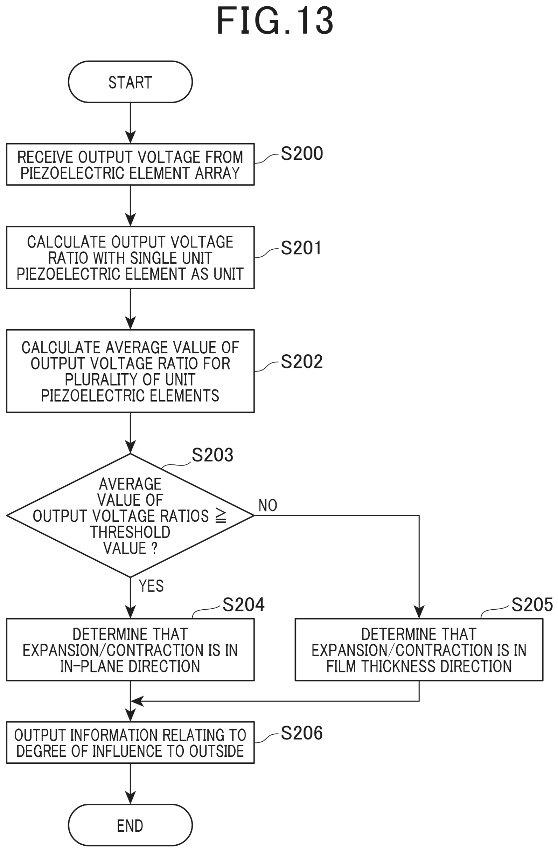

Fig. 13 is a flowchart describing a measurement operation of a measuring device 1 according to a second embodiment of the present invention. The operations of steps S200 to S202 in Fig. 13 are the same as the operations of steps S100 to S102 in Fig. 11.

Then, the control unit 11 determines whether the average value of the output voltage ratio is greater than or equal to a threshold value (step S203). The threshold value is set to an arbitrary value between the maximum value of the output voltage ratio during expansion/contraction in the in-plane direction and the maximum value of the output voltage ratio during expansion/contraction in the film thickness direction. For example, the threshold value is set to an intermediate value between the maximum value of the output voltage ratio during expansion/contraction in the in-plane direction and the maximum value of the output voltage ratio during expansion/contraction in the film thickness direction. In the present embodiment, for example, the threshold value is set to 1.2.

If the average value of the output voltage ratio is greater than or equal to the threshold value (S203 = Yes), the control unit 11 determines that expansion/contraction in the in-plane direction has occurred (step S204). Specifically, the control unit 11 determines that bending or pulling has occurred.

If the average value of the output voltage ratio is less than the threshold value (S203 = No), the control unit 11 determines that expansion/contraction in the film thickness direction has occurred (step S205). Specifically, the control unit 11 determines that pressing has occurred.

Then, the control unit 11 outputs the determination result determined in step S204 and step S205 to the outside (step S206).

According to the second embodiment, it is possible to obtain a determination result indicating whether bending or pulling has occurred or pressing has occurred according to the displacement of the body surface on which the measuring device 1 is worn. The other effects are the same as in the first embodiment.

### [3] Third Embodiment

The third embodiment is another configuration example of the piezoelectric element. In the third embodiment, in the first piezoelectric element 20, the expansion/contraction suppression layer 34 is arranged on the protective layer 38.

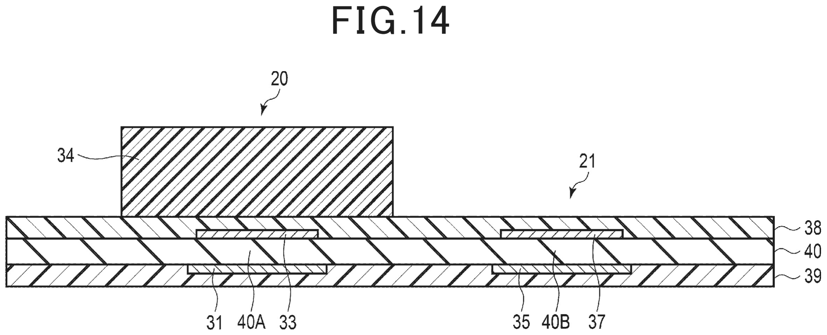

A plan view of the piezoelectric element array 10 according to the third embodiment of the present invention is the same as in Fig. 2. Fig. 14 is a cross-sectional view of a first piezoelectric element 20 and a second piezoelectric element 21 constituting a single unit piezoelectric element 22. Fig. 14 is a cross-sectional view of the unit piezoelectric element 22 of Fig. 2 taken along the X direction.

The piezoelectric element array 10 includes a plurality of first piezoelectric elements 20 and a plurality of second piezoelectric elements 21. An adjacent first piezoelectric element 20 and second piezoelectric element 21 constitute a unit piezoelectric element 22.

The piezoelectric layer 40 is provided in common for the plurality of first piezoelectric elements 20 and the plurality of second piezoelectric elements 21. The piezoelectric layer 40 is provided over the entire piezoelectric element array 10. The configuration of the piezoelectric layer 40 is the same as in the first embodiment.

The first piezoelectric element 20 includes a first lower electrode 31, a partial piezoelectric layer 40A, a first upper electrode 33, and an expansion/contraction suppression layer 34. The first lower electrode 31 is provided on the bottom surface of the partial piezoelectric layer 40A. The first upper electrode 33 is provided on the upper surface of the partial piezoelectric layer 40A.

The protective layer 38 is provided on the upper surface of the partial piezoelectric layer 40A so as to cover the first upper electrode 33. The protective layer 38 is formed by bonding using an adhesive.

The expansion/contraction suppression layer 34 is provided on the protective layer 38. The expansion/contraction suppression layer 34 is provided so as to cover the first upper electrode 33 in a plan view. The area of the expansion/contraction suppression layer 34 is larger than that of the first upper electrode 33. The expansion/contraction suppression layer 34 is composed of a material with a higher modulus of elasticity than the piezoelectric layer 40 and the protective layer 38. The expansion/contraction suppression layer 34 is formed by coating on the protective layer 38. The expansion/contraction suppression layer 34 may also be formed by being bonded to the protective layer 38 using an adhesive.

The configuration of the second piezoelectric element 21 is the same as in the first embodiment.

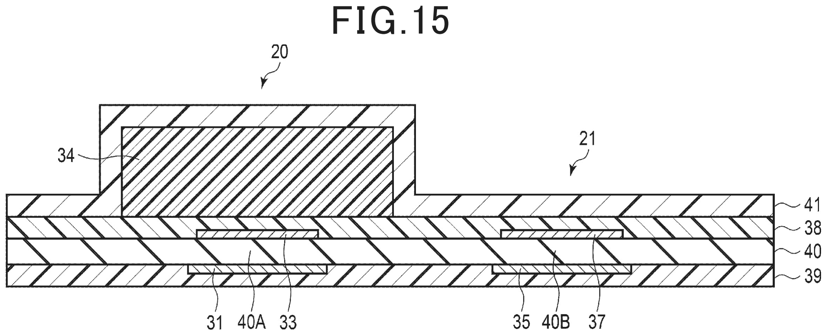

Fig. 15 is a cross-sectional view of a first piezoelectric element 20 and a second piezoelectric element 21 according to a modification. The upper surface of the piezoelectric element array 10 may be further covered with a protective layer.

On the protective layer 38, a protective layer 41 is provided so as to cover the expansion/contraction suppression layer 34. The protective layer 41 is provided over the entire piezoelectric element array 10. The thickness of the protective layer 41 is, for example, about 50 µm. The protective layer 41 is formed by bonding using an adhesive. The material of the protective layer 41 is the same as the material of the protective layer 38.

The rest of the configuration is the same as in the first embodiment. Similarly, in the measuring device 1 according to the third embodiment, it is possible to perform the same operations as in the first embodiment.

### [4] Fourth Embodiment

The fourth embodiment is another configuration example of the piezoelectric element. The fourth embodiment includes a substrate that supports the entire piezoelectric element array 10, and further, a piezoelectric layer is provided for each piezoelectric element.

### [4-1] Configuration of Piezoelectric Element Array 10

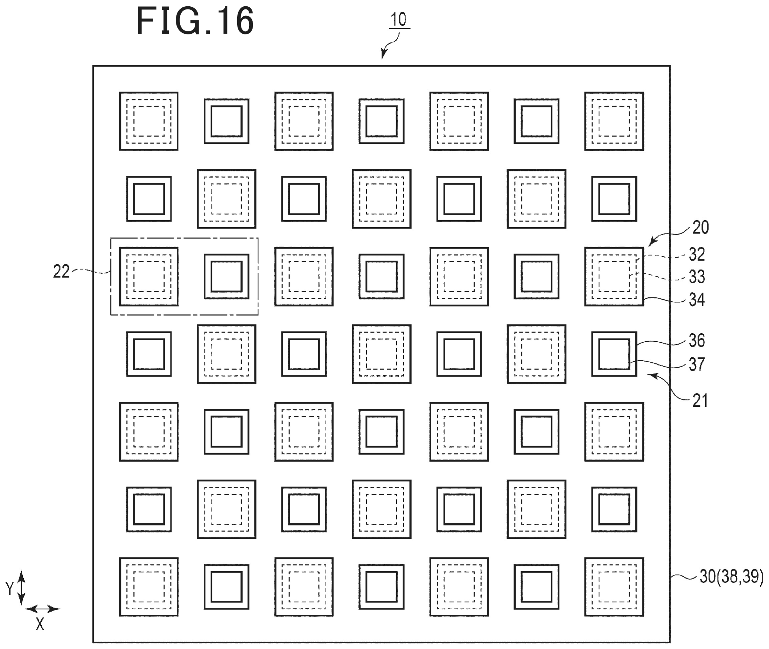

Fig. 16 is a plan view of the piezoelectric element array 10 according to the fourth embodiment of the present invention. The piezoelectric element array 10 includes a plurality of first piezoelectric elements 20 and a plurality of second piezoelectric elements 21. An adjacent first piezoelectric element 20 and second piezoelectric element 21 constitute a unit piezoelectric element 22. The arrangement example of the plurality of first piezoelectric elements 20 and the plurality of second piezoelectric elements 21 is the same as in the first embodiment.

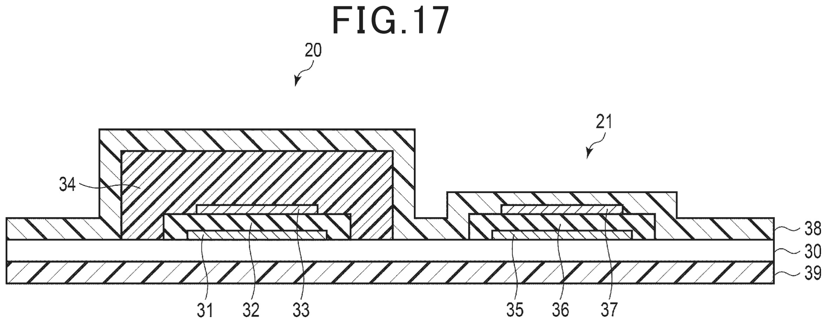

Fig. 17 is a cross-sectional view of a first piezoelectric element 20 and a second piezoelectric element 21 constituting a single unit piezoelectric element 22. Fig. 17 is a cross-sectional view of the unit piezoelectric element 22 of Fig. 16 taken along the X direction.

The piezoelectric element array 10 includes a substrate 30. The substrate 30 is provided in common for the plurality of first piezoelectric elements 20 and the plurality of second piezoelectric elements 21. The substrate 30 is provided over the entire piezoelectric element array 10. The substrate 30 is composed of an insulating material, and is composed of a resin having flexibility and/or an expansion/contraction property. It is desirable that the substrate 30 is a resin with high moisture permeability. The substrate 30 is composed of, for example, a polyurethane resin, a polyolefin resin, a polystyrene resin, or a polyester resin. The thickness of the substrate 30 is, for example, about 100 µm.

The first piezoelectric element 20 includes a first lower electrode 31, a first piezoelectric layer 32, a first upper electrode 33, and an expansion/contraction suppression layer 34.

The first lower electrode 31 is provided on the substrate 30. The material and shape of the first lower electrode 31 are the same as in the first embodiment. The thickness of the first lower electrode 31 is, for example, about 200 nm.

The first piezoelectric layer 32 is provided on the first lower electrode 31. The first piezoelectric layer 32 may have, for example, a square shape. The first piezoelectric layer 32 has an area larger than that of the first lower electrode 31, and covers the entire first lower electrode 31. The material of the first piezoelectric layer 32 is the same as that of the piezoelectric layer 40 described in the first embodiment. More preferably, the first piezoelectric layer 32 is composed of a P(VDF/TrFE)-containing material, which may be formed using a printing method. The first piezoelectric layer 32 preferably has an expansion/contraction property. The thickness of the first piezoelectric layer 32 is, for example, about 2 µm.

The first upper electrode 33 is provided on the first piezoelectric layer 32. The material and shape of the first upper electrode 33 are the same as in the first embodiment. The thickness of the first upper electrode 33 is, for example, about 10 µm.

The expansion/contraction suppression layer 34 is provided on the first upper electrode 33, the first piezoelectric layer 32, and the substrate 30. The expansion/contraction suppression layer 34 has, for example, a square shape. The area of the expansion/contraction suppression layer 34 is larger than the area of the first piezoelectric layer 32. The expansion/contraction suppression layer 34 is configured to cover the first lower electrode 31, the first piezoelectric layer 32, and the first upper electrode 33, and the peripheral portion is in contact with the substrate 30.

The expansion/contraction suppression layer 34 is composed of a material with a higher modulus of elasticity than the first piezoelectric layer 32. Furthermore, the expansion/contraction suppression layer 34 is composed of a material with a higher modulus of elasticity than the protective layer 38, which will be described later. The expansion/contraction suppression layer 34 is formed by coating on the substrate 30, the first piezoelectric layer 32, and the first upper electrode 33. The expansion/contraction suppression layer 34 may also be formed by being bonded to the substrate 30, the first piezoelectric layer 32, and the first upper electrode 33 using an adhesive.

The second piezoelectric element 21 includes a second lower electrode 35, a second piezoelectric layer 36, and a second upper electrode 37.

The second lower electrode 35 is provided on the substrate 30. The second piezoelectric layer 36 is provided on the second lower electrode 35. The second upper electrode 37 is provided on the second piezoelectric layer 36. The configuration of the second lower electrode 35, the second piezoelectric layer 36, and the second upper electrode 37 is the same as that of the first lower electrode 31, the first piezoelectric layer 32, and the first upper electrode 33. The areas and thicknesses of the second lower electrode 35, the second piezoelectric layer 36, and the second upper electrode 37 are the same as those of the first lower electrode 31, the first piezoelectric layer 32, and the first upper electrode 33. Note that the materials, areas, and thicknesses of the second lower electrode 35, the second piezoelectric layer 36, and the second upper electrode 37 may be different from those of the first lower electrode 31, the first piezoelectric layer 32, and the first upper electrode 33.

The protective layer 38 is provided on the first piezoelectric element 20 and the second piezoelectric element 21. The protective layer 38 is configured to cover the first piezoelectric element 20 and the second piezoelectric element 21. The protective layer 38 is provided over the entire piezoelectric element array 10. The material of the protective layer 38 is the same as in the first embodiment. The protective layer 38 is formed by bonding using an adhesive.

The protective layer 39 is provided on the bottom surface of the substrate 30. The protective layer 39 is provided over the entire surface of the substrate 30. The material of the protective layer 39 is the same as that of the protective layer 38. The thickness of the protective layer 39 is, for example, about 50 µm. The protective layer 39 is formed by bonding using an adhesive.

### [4-2] Operation

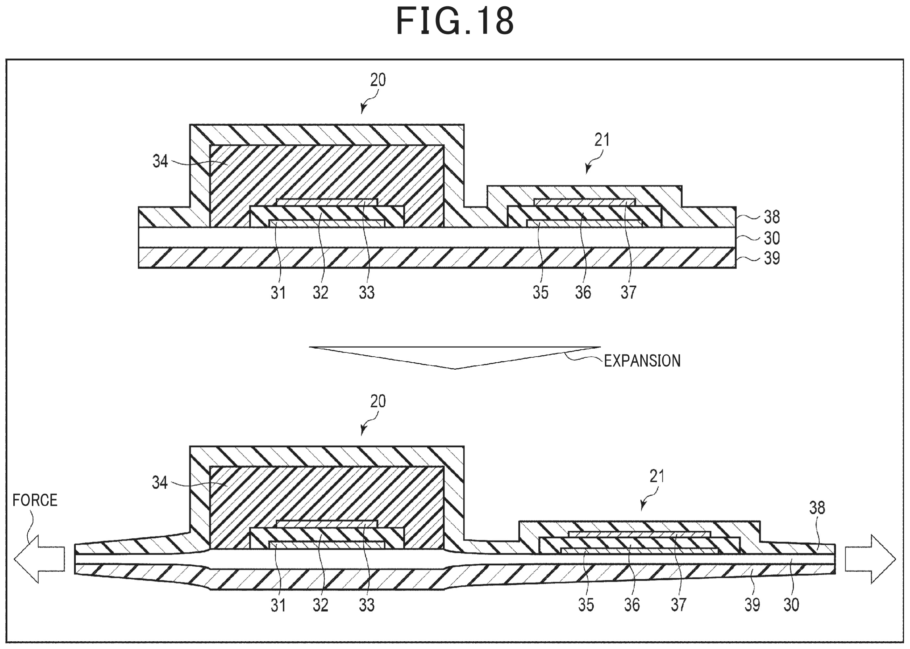

Fig. 18 is a diagram describing a state where the piezoelectric element array 10 is expanded in the in-plane direction. Fig. 18 shows a single unit piezoelectric element 22 (the first piezoelectric element 20 and the second piezoelectric element 21) extracted for illustration. For example, when bending occurs in the piezoelectric element array 10, the piezoelectric element array 10 is expanded in the in-plane direction.

The substrate 30, the protective layer 38, and the protective layer 39 have flexibility and/or an expansion/contraction property. When bending occurs in the piezoelectric element array 10, the substrate 30 is expanded in the in-plane direction. Along with the expansion of the substrate 30, the second piezoelectric element 21 (specifically, the second piezoelectric layer 36) is expanded in the in-plane direction. As a result, because the second piezoelectric layer 36 is expanded in the in-plane direction, the output voltage of the second piezoelectric element 21 becomes larger.

On the other hand, the first piezoelectric element 20 is provided with the expansion/contraction suppression layer 34, which has a high modulus of elasticity. Therefore, the expansion of the first piezoelectric element 20 (specifically, the first piezoelectric layer 32) is suppressed compared to the second piezoelectric element 21. When the expansion/contraction suppression layer 34 is provided, because the modulus of elasticity of the expansion/contraction suppression layer 34 is high, most of the stress due to in-plane expansion is applied to regions other than the region where the expansion/contraction suppression layer 34 is formed. As a result, the first piezoelectric element 20 has a smaller output voltage than the second piezoelectric element 21. That is, in the present embodiment, the output voltage ratio during expansion/contraction becomes less than 1.

Even when the output voltage ratio during expansion/contraction is less than 1, the determination method based on the output voltage ratio is the same as when the output voltage ratio during expansion/contraction is greater than 1. When the determination is made using the degree of influence, the smaller the output voltage ratio, the greater the degree of influence from expansion/contraction. Furthermore, when setting a reference value (threshold value), the value is determined such that a value greater than or equal to the reference value is due to pressing, and a value less than the reference value is due to expansion/contraction.

Similarly, in the fourth embodiment, the same effects as in the first embodiment can be obtained.

### [5] Fifth Embodiment

The fifth embodiment is an example relating to the arrangement of the plurality of first piezoelectric elements 20 and the plurality of second piezoelectric elements 21.

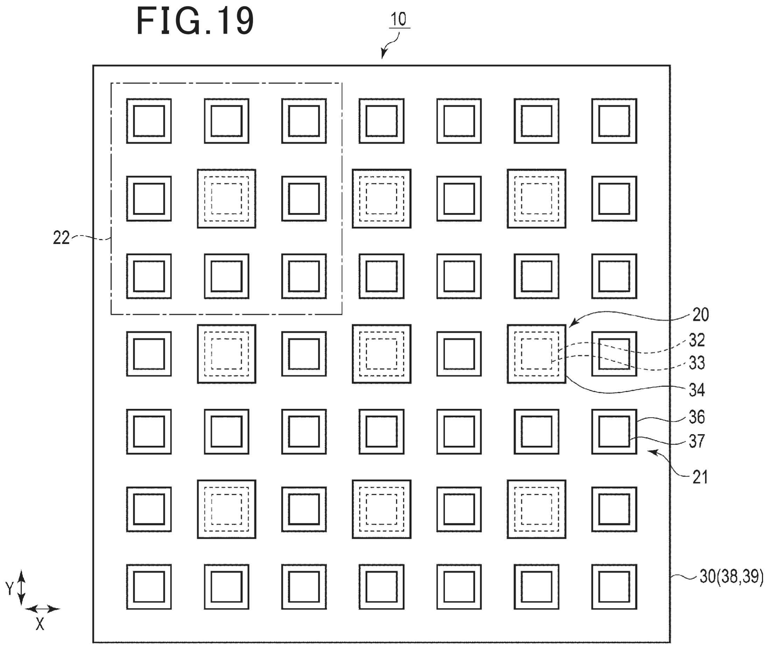

Fig. 19 is a plan view of the piezoelectric element array 10 according to the fifth embodiment of the present invention. The piezoelectric element array 10 includes a plurality of first piezoelectric elements 20 and a plurality of second piezoelectric elements 21. The plurality of first piezoelectric elements 20 and the plurality of second piezoelectric elements 21 are arranged in a matrix overall. The respective configurations of the first piezoelectric element 20 and the second piezoelectric element 21 are the same as in the fourth embodiment.

The number of second piezoelectric elements 21 is set to be greater than the number of first piezoelectric elements 20. Specifically, eight second piezoelectric elements 21 are arranged around one first piezoelectric element 20 (above, below, left, right, and diagonally). Every other element arranged in the X direction and Y direction is one of the plurality of first piezoelectric elements 20. In other words, the plurality of first piezoelectric elements 20 and the plurality of second piezoelectric elements 21 are alternately arranged in each of the X direction, the Y direction, and the diagonal directions.

A single first piezoelectric element 20 and the eight surrounding second piezoelectric elements 21 constitute a unit piezoelectric element 22. The adjacent unit piezoelectric elements 22 in the X direction share three second piezoelectric elements 21 in the overlapping portions. Similarly, the adjacent unit piezoelectric elements 22 in the Y direction share three second piezoelectric elements 21 in the overlapping portions.

The output voltage ratio of the unit piezoelectric element 22 is calculated as "(first output voltage of the single first piezoelectric element 20) / (average value of the second output voltages of the eight surrounding second piezoelectric elements 21)", using the first output voltage of the single first piezoelectric element 20 and the average value of the second output voltages of the eight surrounding second piezoelectric elements 21.

Note that the plurality of first piezoelectric elements 20 and the plurality of second piezoelectric elements 21 may be arranged such that adjacent unit piezoelectric elements 22 in the X and Y directions do not share the second piezoelectric elements 21. In the case of such a modification, every three elements arranged in the X direction and Y direction is one of the plurality of first piezoelectric elements 20.

According to the fifth embodiment, because the area occupied by the expansion/contraction suppression layers 34 with a high modulus of elasticity becomes smaller, the substrate 30 can expand/contract more easily. Therefore, it is possible to configure a piezoelectric element array 10 that has an enhanced flexibility and expansion/contraction property. The other effects are the same as in the first embodiment. It is also possible to apply the first to third embodiments to the fifth embodiment.

The present invention is not limited to the embodiments described above, but may be modified in various ways when implemented, without departing from the spirit of the present invention. Furthermore, the embodiments may be appropriately combined when implemented, and in this case, a combination of the advantageous effects is obtained. The embodiments described above include various inventions, and various inventions can be extracted from combinations selected from a plurality of disclosed constituent elements. For example, even if some constituent elements are eliminated from all the constituent elements disclosed in the embodiments, configurations with these constituent elements eliminated can be interpreted to be within the scope of the invention as long as the problems can be solved and the advantageous effects can be obtained.

### [Reference Signs List]

- 1: Measuring device,
- 10: Piezoelectric element array,
- 11: Control unit,
- 12: Storage unit,
- 13: Power supply,
- 20: First piezoelectric element,
- 21: Second piezoelectric element,
- 22: Unit piezoelectric element,
- 30: Substrate,
- 31: First lower electrode,
- 32: First piezoelectric layer,
- 33: First upper electrode,
- 34: Expansion/contraction suppression layer,
- 35: Second lower electrode,
- 36: Second piezoelectric layer,
- 37: Second upper electrode,
- 38: Protective layer,
- 39: Protective layer,
- 40: Piezoelectric layer,
- 40A, 40B: Partial piezoelectric layer,
- 41: Protective layer.

## Claims

1. A measuring device comprising:
a first piezoelectric element and a second piezoelectric element; and
a piezoelectric layer provided in common for the first piezoelectric element and the second piezoelectric element, and which includes a first partial piezoelectric layer and a second partial piezoelectric layer; wherein
the first piezoelectric element includes a first lower electrode, the first partial piezoelectric layer provided on the first lower electrode, a first upper electrode provided on the first partial piezoelectric layer, and an expansion/contraction suppression layer provided on the first partial piezoelectric layer so as to cover the first upper electrode,
the second piezoelectric element includes a second lower electrode, the second partial piezoelectric layer provided on the second lower electrode, and a second upper electrode provided on the second partial piezoelectric layer, and
the expansion/contraction suppression layer has a higher modulus of elasticity than the piezoelectric layer.

2. The measuring device according to claim 1, further comprising
a first protective layer provided on the piezoelectric layer so as to cover the expansion/contraction suppression layer and the second upper electrode.

3. A measuring device comprising:
a first piezoelectric element and a second piezoelectric element; and
a piezoelectric layer provided in common for the first piezoelectric element and the second piezoelectric element, and which includes a first partial piezoelectric layer and a second partial piezoelectric layer, wherein
the first piezoelectric element includes a first lower electrode, the first partial piezoelectric layer provided on the first lower electrode, a first upper electrode provided on the first partial piezoelectric layer, a first protective layer provided on the first partial piezoelectric layer so as to cover the first upper electrode, and an expansion/contraction suppression layer provided on the first protective layer,
the second piezoelectric element includes a second lower electrode, the second partial piezoelectric layer provided on the second lower electrode, and a second upper electrode provided on the second partial piezoelectric layer,
the first protective layer is formed so as to further cover the second upper electrode, and
the expansion/contraction suppression layer has a higher modulus of elasticity than the piezoelectric layer.

4. The measuring device according to claim 1 or 3, wherein
the piezoelectric layer has flexibility or an expansion/contraction property.

5. The measuring device according to claim 1 or 3, wherein
the piezoelectric layer is formed of an organic piezoelectric material having an expansion/contraction property.

6. The measuring device according to claim 1 or 3, further comprising
a second protective layer provided on a bottom surface of the piezoelectric layer so as to cover the first lower electrode and the second lower electrode.

7. A measuring device comprising:
a substrate;
a first piezoelectric element and a second piezoelectric element provided on the substrate; and
a protective layer covering the first piezoelectric element and the second piezoelectric element, wherein
the first piezoelectric element includes a first lower electrode provided on the substrate, a first piezoelectric layer provided on the first lower electrode, a first upper electrode provided on the first piezoelectric layer, and an expansion/contraction suppression layer provided on the substrate so as to cover the first upper electrode, the first piezoelectric layer, and the first upper electrode,
the second piezoelectric element includes a second lower electrode provided on the substrate, a second piezoelectric layer provided on the second lower electrode, and a second upper electrode provided on the second piezoelectric layer, and
the expansion/contraction suppression layer has a higher modulus of elasticity than the first piezoelectric layer and the protective layer.

8. The measuring device according to claim 7, wherein
the substrate has flexibility or an expansion/contraction property.

9. The measuring device according to claim 7, wherein
the first piezoelectric layer and the second piezoelectric layer are formed of an organic piezoelectric material having an expansion/contraction property.

10. The measuring device according to any one of claims 1, 3, and 7, wherein
a plurality of the first piezoelectric elements and second piezoelectric elements are arranged in an array.

11. The measuring device according to any one of claims 1, 3, and 7, further comprising
a control unit that calculates an output voltage ratio between the first piezoelectric element and the second piezoelectric element, and based on the output voltage ratio, determines a degree of influence indicating whether there is expansion/contraction in an in-plane direction or expansion/contraction in a film thickness direction.

12. The measuring device according to any one of claims 1, 3, and 7, further comprising
a control unit that calculates an output voltage ratio between the first piezoelectric element and the second piezoelectric element, and based on a comparison result between the output voltage ratio and a threshold value, determines whether there is expansion/contraction in an in-plane direction or expansion/contraction in a film thickness direction.
